# EUROPÄISCHE PATENTSCHRIFT

(11) **EP 3 366 714 B1**
(45) Veröffentlichungstag und Bekanntmachung des Hinweises auf die Patenterteilung: **30.03.2022**
(21) Anmeldenummer: 18158133.1
(22) Anmeldetag: 22.02.2018
(51) Int. Cl.: B27N 1/00, C07D 307/50, B27N 3/00, B32B 21/04, C08G 8/04, C08L 61/06, C09J 161/06

(54) **VERFAHREN ZUR HERSTELLUNG THERMISCH HÄRTBARER PHENOLHARZE SOWIE DURCH DAS VERFAHREN ERHÄLTLICHE PHENOLHARZE**
METHOD FOR PRODUCING THERMALLY CURABLE PHENOL RESINS AND PHENOL RESIN OBTAINABLE BY THE METHOD
PROCÉDÉ DE FABRICATION DE RÉSINE PHÉNOLIQUE THERMODURCISSABLE AINSI QUE RÉSINE PHÉNOLIQUE OBTENUE SELON LE PROCÉDÉ

(30) Priorität: 27.02.2017 EP 17158247; 27.02.2017 EP 17158248; 27.02.2017 EP 17158249
(43) Veröffentlichungstag der Anmeldung: 29.08.2018
(73) Patentinhaber: AVA Biochem AG, 6304 Zug (CH)
(72) Erfinder: Mortato, Mariangela, 4051 Basel Stadt (CH); Krawielitzki, Stefan, 6343 Holzhäusern (CH); Badoux, François, 6314 Unterägeri (CH); Holmes, Christopher, 4208 Nunningen (CH); Ghorbani, Masoumeh, 3006 Bern (CH); Sanglard, Marion, 3014 Bern (CH); Frei, Reto, 2533 Evilard (CH)
(74) Vertreter: Christ, Niko

(56) Entgegenhaltungen:
- US-A- 2 776 948
- US-A- 2 937 158
- US-A- 4 524 164

## Beschreibung

Die vorliegende Erfindung betrifft ein Verfahren zur Herstellung von thermisch härtbaren Phenolharzen Im Besonderen betrifft die Erfindung ein Verfahren zur Herstellung von thermisch härtbaren Phenolharzen, welche wenigstens ein durch Polykondensation von phenolischen Verbindungen mit HMF erhaltenes Polykondensationsprodukt umfassen

Thermisch härtbare Harze werden vorzugsweise durch die Polykondensation von phenolischen Verbindungen und/oder Aminoplastbildnern mit reaktiven Carbonylverbindungen, insbesondere Aldehyden, gewonnen. Beispielhaft seien Aminoharze mit den Aminoplastbildnern Harnstoff, Melamin und Dicyandiamid, Phenolharze oder Amino-Phenol-Harze genannt. Die Harze zeichnen sich im Allgemeinen durch gute Verarbeitungseigenschaften wie eine hohe Reaktivität aus. Durch anschließende Aushärtung der Harze erhält man einen duroplastischen Werkstoff.

Zur Herstellung von Holzverbundwerkstoffen werden die Harze üblicherweise mit zerkleinertem Holz, beispielsweise mit Holzspänen oder Holzfasern vermischt, wonach man sie bei erhöhten Temperaturen verpresst, wobei die Harze unter Vernetzung aushärten.

Aufgrund seiner hohen Reaktivität kommt für die Polykondensation vorwiegend Formaldehyd zum Einsatz. Zur Förderung der Umsetzung wird häufig mit einem Überschuss an Formaldehyd gearbeitet, sodass die Harze einen hohen Gehalt an freiem Formaldehyd aufweisen. Die Formaldehydemission der Harze ist daher hoch.

Nachteilig dabei ist die von Formaldehyd ausgehende Gesundheitsgefährdung, sodass die Verwendung von Formaldehyd auch zunehmend reguliert wird.

Aufgrund des Gefährdungspotenzials ist man seit Jahren darum bemüht, den Gehalt an Formaldehyd zu reduzieren. Eine Maßnahme hierbei ist, Formaldehyd bei der Herstellung der Harze durch andere reaktive Verbindungen zu ersetzen. 5-Hydroxymethylfurfural (HMF) wurde bereits als ein vielversprechender Kandidat hierfür identifiziert, da dieses eine Fähigkeit besitzt, quervernetzende Bindungen auszubilden, schwer-flüchtig sowie praktisch ungiftig ist und aus erneuerbaren Rohstoffen gewonnen werden kann.

In der Fachzeitschrift European Journal of Wood Products wird ein HMFmodifiziertes Harnstoff-Formaldehyd-Harz beschrieben, bei dessen Herstellung bis zu etwa 30 Gew.% des Formaldehyds durch gereinigtes, kristallines HMF ersetzt wurden (N. Esmaeili et al., DOI 10.1007/s0017-016-1072-8). Mit diesem Harz hergestellte Spanplatten zeigen eine innere Bindung (IB) von ≥ 0,35 N/mm², wie sie zur Erfüllung des Mindeststandards für Platten in Innenräumen gemäß der europäischen Norm NEN EN 319 derzeit benötigt werden. Nachteilig ist jedoch, dass das Harz und daraus hergestellte Spanplatten noch erhebliche Mengen an toxischem Formaldehyd enthalten.

Die US 2 776 948 A offenbart die Herstellung von Kunstharzen auf der Basis von HMF und phenolischen Verbindungen. Das hierbei eingesetzte HMF liegt in einem Die US 2 776 948 A offenbart die Herstellung von Kunstharzen auf der Basis von HMF und phenolischen Verbindungen. Das hierbei eingesetzte HMF liegt in einem Hydrolysat vor, welches aus hexosehaltigem Material wie säureimprägniertem Holz durch Dampfdruckhydrolyse hergestellt wurde. Zur Aushärtung der Kunstharze oder zur Herstellung von Fieberglasmatten wird den Kunstharzen wiederum toxisches Formaldehyd zugesetzt.

Die US 2 937 158 betrifft die Herstellung von Harzen aus Phenolen und Hydrolyseprodukten von Lignocellulose. Das lignocellulosehaltige Material wird bei hoher Temperatur und unter Dampfdruck mit einer verdünnten Säurelösung unter Bedingungen imprägniert, die neben einer wässrigen, HMF-haltigen Phase eine halbfeste, harzartige Reaktionsmasse liefern. In der Reaktionsmasse enthaltene Hydrolyseprodukte werden mit oxygenierten organischen Lösungsmitteln extrahiert und mit Phenolen bei einem pH-Wert von etwa 10,5 bis etwa 11,5 umgesetzt. Die erhaltenen Kunstharze werden nicht thermisch gehärtet.

Die US 4 524 164 A beschreibt formaldehydfreie, thermisch härtbare Harze, welche als Bindemittel für lignocellulosehaltiges Material zur Herstellung von Sperrholz- und Spanplatten dienen. Zunächst werden hierzu zuckerhaltige Lösungen unter sauren Bedingungen und in Gegenwart eines Metallkatalysators bei Temperaturen von 50°C bis 200°C zu einem flüssigen, schmelzbaren Harz umgesetzt. Als Vernetzungsmittel für die Zucker und Zuckerabbauprodukte werden dem Ansatz phenolische Verbindungen oder Harnstoff zugegeben. Von Nachteil ist, dass unter diesen Bedingungen hergestellte Harze erst durch den Zusatz eines Härters thermisch härtbar gemacht werden.

Die Aufgabe der vorliegenden Erfindung besteht demnach in der Beseitigung der oben genannten Nachteile.

Dies gelingt nach einem ersten Aspekt der vorliegenden Erfindung durch ein Verfahren zur Herstellung thermisch härtbarer Phenolharze gemäß den Merkmalen des Anspruchs 1.

Das Verfahren zur Herstellung von thermisch härtbaren Phenolharzen enthält den Schritt der Umsetzung einer polykondensationsfähigen phenolischen Verbindung mit 5-Hydroxymethylfurfural (HMF) unter zur Bildung von Polykondensationsprodukten führenden Bedingungen und ist dadurch gekennzeichnet, dass das HMF mindestens ein HMF-Oligomer umfasst, und der Umsetzungsschritt bei pH-Werten von über 7 für mehr als 60 Minuten durchgeführt wird. Es wurde gefunden, dass unter den erfindungsgemäßen Bedingungen auf Formaldehyd und Härter sowohl bei der Herstellung als auch der Weiterverarbeitung der Phenolharze vollkommen verzichtet werden kann und thermisch härtbare

Phenolharze erhalten werden können, welche eine zu Phenol-Formaldehyd-Harzen vergleichbare Bindungsstärke aufweisen, sofern für die Polykondensation HMF eingesetzt wird, das HMF-Oligomere enthält.

Das Auftreten von wasserlöslichen linearen und verzweigten HMF-Oligomeren in Lösungen von HMF ist beispielweise aus der DE 10 2014 112 240 A1 bekannt. Die HMF-Oligomere entstehen unter anderem bei der Herstellung von HMF aus Kohlenhydraten und kohlenhydrathaltiger Biomasse unter hydrothermalen Bedingungen und können durch NMR-, IR- und Massen-Spektroskopie nachgewiesen werden. Die Bildung der HMF-Oligomere kann beispielsweise auch mittels HPLC-Analysen verfolgt werden.

Als HMF-Oligomere im Sinne der vorliegenden Erfindung werden im Unterschied zu HMF-Monomeren Verbindungen aus mindestens zwei verknüpften HMF-Einheiten/Monomeren bezeichnet. Üblicherweise versteht man unter HMF-Oligomeren Verbindungen mit einem Molekulargewicht von bis zu 3000 g/mol. Für das Verfahren eignen sich insbesondere HMF-Oligomere mit einem niedrigen Molekulargewicht, die unter den gewählten Umsetzungsbedingungen in dem gewählten Lösungsmittel löslich oder zumindest dispergiert vorliegen. Der Übergang zwischen gelöster und dispergierter Form kann hierbei fließend sein, sodass in der vorliegenden Erfindung diesbezüglich nicht unterschieden werden soll.

Bislang bekannte oligomere Verbindungen aus HMF resultieren aus der Verknüpfung von Aldehyd- und/oder Hydroxylgruppen einzelner HMF-Monomere oder einzelner Monomere mit aus HMF-Monomeren bestehenden HMF-Oligomeren. Die HMF-Monomere stellen schließlich die Einheiten der gebildeten HMF-Oligomere dar. Die HMF-Oligomere sind linear, mehr oder weniger stark verzweigt und weisen Ether-, Hemiacetal- und/oder Acetal-Bindungen auf. HMF-Oligomere werden sowohl unter sauren als auch unter basischen Bedingungen gebildet.

Lineare HMF-Oligomere enthalten üblicherweise Strukturelemente, die durch Ether-Bindungen verknüpfte Einheiten des Typs 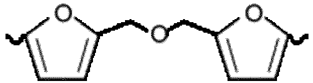 und/oder durch Bildung von Halbacetalen verknüpfte Einheiten des Typs 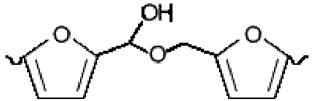 umfassen. Verzweigte HMF-Oligomere können zudem Strukturelemente mit unter Acetalbildung verknüpften Einheiten des Typs 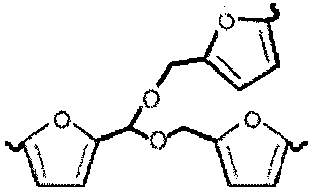 enthalten. Die geschwungenen Linien deuten dabei an, dass es sich bei den dargestellten Strukturelementen um einen Ausschnitt aus einem HMF-Oligomer handelt. Ein HMF-Oligomer weist in der Regel mehrere gleiche oder unterschiedliche Strukturelemente der angegebenen Typen auf. Endständige HMF-Einheiten werden von Aldehyd- oder Hydroxymethylgruppen begrenzt.

Für den Fachmann ist es dabei selbstverständlich, dass das mindestens eine HMF-Oligomer in einer Mischung aus HMF-Oligomeren verschiedener Länge und/oder verschiedenen Vernetzungsgrads vorliegen kann. Es ist zudem möglich, durch die Auswahl eines HMF-Oligomers oder durch die Auswahl einer Kombination unterschiedlicher HMF-Oligomere, die Eigenschaften des resultierenden Phenolharzes gezielt auf den technischen Verwendungszweck abzustimmen.

Die Polykondensation nimmt man in an sich bekannter Weise vor. Für die Umsetzung geeignete Lösungsmittel sind dem Fachmann grundsätzlich bekannt. Unter geeigneten Lösungsmitteln werden erfindungsgemäß jegliche flüssige Mittel verstanden, in denen sich Polykondensationsprodukte bilden können. Bevorzugt wird die Umsetzung in einem wässrigen Lösungsmittel durchgeführt. Insbesondere kann der Umsetzungsschritt in Wasser durchgeführt werden.

Der alkalische pH-Wert kann durch geeignete Basen zu Beginn des Umsetzungsschritts eingestellt werden. Ohne den Gegenstand der Erfindung beschränken zu wollen, werden beispielhaft Alkalihydroxide, Erdalkalihydroxide, Natriumcarbonat, Ammoniak und tertiäre Amine als geeignete Basen genannt, wobei insbesondere Natriumhydroxid zur Einstellung des alkalischen pH-Wertes geeignet ist.

Es wurde gefunden, dass Reaktionszeiten im Umsetzungsschritt von über 60 Minuten bei basischen pH-Werten mit mindestens einem HMF-Oligomer umfassendem HMF zu Phenolharzen führen, die eine hohe Bindungsstärke aufweisen.

Bevorzugt wird der Umsetzungsschritt solange durchgeführt, bis die Lösung eine gewünschte Viskosität erreicht hat oder die Reaktion abgeschlossen ist. Gewünschte Viskositäten liegen üblicherweise zwischen 100 mPa.s und 1200 mPa.s. Weiter bevorzugt wird der Umsetzungsschritt solange durchgeführt, bis die Lösung eine Viskosität von über 200 mPa.s erreicht hat, besonders bevorzugt bis die Lösung eine Viskosität von über 800 mPa.s, weiter besonders bevorzugt bis die Lösung eine Viskosität von über 850 mPa.s erreicht hat.

Nach einer vorteilhaften Ausgestaltung des Verfahrens wird der Umsetzungsschritt bei pH-Werten von 7,5 bis 14, bevorzugt bei pH-Werten von 9 bis 14, weiter bevorzugt bei pH-Werten von 9,5 bis 13, besonders bevorzugt bei pH-Werten von 9,5 bis 12,5, weiter besonders bevorzugt bei Werten von 9,8 bis 12 durchgeführt.

Nach einer weiteren vorteilhaften Ausgestaltung des Verfahrens wird der Umsetzungsschritt für mehr als 60 Minuten bis 15 Stunden, für 65 Minuten bis 15 Stunden, für 80 Minuten bis 15 Stunden, für 100 Minuten bis 10 Stunden durchgeführt. Weiter bevorzugt wird der Umsetzungsschritt für 2 bis 7 Stunden durchgeführt, besonders bevorzugt für 3 bis 7 Stunden, insbesondere 5 bis 6,5 Stunden, weiter besonders bevorzugt für 5 Stunden bis 6 Stunden.

Nach einer weiteren vorteilhaften Ausgestaltung des Verfahrens wird der Umsetzungsschritt bei Temperaturen im Bereich von 40°C bis 170°C, bevorzugt im Bereich von 50°C bis 150°C, weiter bevorzugt im Bereich von 60°C bis 110°C, besonders bevorzugt im Bereich von 70°C bis 110°C, weiter besonders bevorzugt im Bereich von 85°C bis 110°C durchgeführt. Prinzipiell kann die Temperatur zur Durchführung des Verfahrens in einem breiten Bereich variiert werden. Es wurde jedoch beobachtet, dass die Umsetzung bei Temperaturen unter 40°C sehr langsam abläuft. Werden Temperaturen von über 60°C eingesetzt, verläuft die Umsetzung deutlich schneller. Dies war unerwartet, da man bislang davon ausging, dass bereits ab Temperaturen über 50°C eine zunehmende Zersetzung von HMF stattfindet.

Nach einer weiteren vorteilhaften Ausgestaltung des Verfahrens wird der Umsetzungsschritt in mindestens zwei Stufen vorgenommen, wobei eine erste Stufe des Umsetzungsschritts bei Temperaturen im Bereich von 40°C bis 80°C durchgeführt wird, und eine weitere Stufe des Umsetzungsschritts bei Temperaturen durchgeführt wird, die höher als die Temperaturen der ersten Stufe sind. Eine mehrstufige Durchführung des Verfahrens ist aus ökonomischen Gesichtspunkten vorteilhaft. Es wurde beobachtet, dass der Umsetzungsschritt in einer frühen Phase bei einer geringeren Temperatur durchgeführt werden kann, als in einer späteren Phase. Hierdurch genügt ein geringeres Aufheizen in der ersten Stufe des Umsetzungsschritts und es werden Phenolharze mit einer sehr guten Bindungsstärke erhalten. Eine zweistufige Durchführung des Verfahrens ist bevorzugt.

Nach einer weiteren vorteilhaften Ausgestaltung des Verfahrens wird der Umsetzungsschritt in einer ersten Stufe für 50 bis 100 Minuten bei Temperaturen im Bereich von 40°C bis 80°C und in einer zweiten Stufe für 15 Minuten bis 14 Stunden bei Temperaturen im Bereich von 85°C bis 170°C durchgeführt. Bevorzugt wird der Umsetzungsschritt in der ersten Stufe im Bereich von 50°C bis 75°C, weiter bevorzugt im Bereich von 60°C bis 68°C, besonders bevorzugt im Bereich von 63°C bis 67°C, weiter besonderes bevorzugt bei einer Temperatur bis 65°C durchgeführt. Bevorzugt wird der Umsetzungsschritt in der ersten Stufe für 50 bis 90 Minuten, weiter bevorzugt für 50 bis 80 Minuten, besonders bevorzugt für 55 bis 70 Minuten durchgeführt. Bevorzugt wird der Umsetzungsschritt in der zweiten Stufe bei Temperaturen im Bereich von 85°C bis 150°C, weiter bevorzugt von 85°C bis 110°C, besonders bevorzugt von 85 bis 100°C, weiter besonders bevorzugt von 87°C bis 93°C, insbesondere bevorzugt bei 90°C durchgeführt. Bevorzugt wird der Umsetzungsschritt in der zweiten Stufe für 60 Minuten bis 7 Stunden, weiter bevorzugt für 3 bis 6 Stunden, besonders bevorzugt für 3,5 bis 5,5 Stunden, weiter besonders bevorzugt für 4,5 bis 5,5 Stunden durchgeführt.

Nach einer weiteren vorteilhaften Ausgestaltung des Verfahrens beträgt das molare Verhältnis der eingesetzten HMF-Menge zu der Gesamtmenge an phenolischer Verbindung 0,5:1 bis 4:1, bevorzugt beträgt das molare Verhältnis 1,3:1 bis 3:1, weiter bevorzugt beträgt das molare Verhältnis 1:1 bis 2,7:1, besonders bevorzugt beträgt das molare Verhältnis 1,5:1 bis 2,6:1, weiter besonders bevorzugt 2:1 bis 2,5:1. Prinzipiell kann das molare Verhältnis der eingesetzten HMF-Menge zu der Gesamtmenge an phenolischer Verbindung über einen breiten Bereich variiert werden. Ein molarer Überschuss von HMF ist insbesondere vorteilhaft, da damit der Gehalt an Restmonomeren der phenolischen Verbindung im Phenolharz vermindert werden kann. Ein für die jeweilige phenolische Verbindung zur Durchführung des erfindungsgemäßen Verfahrens geeignetes molares Verhältnis lässt sich für den Fachmann auf der Basis der molaren Masse von HMF (126,11 g/mol) rechnerisch leicht ermitteln, indem die molare Masse als Berechnungsgrundlage dient, ohne Berücksichtigung, welcher Anteil der HMF-Menge tatsächlich als Monomer vorliegt oder in HMF-Oligomere eingebunden ist.

Nach einer weiteren vorteilhaften Ausgestaltung des Verfahrens beträgt der Anteil an HMF-Oligomer 0,05 Gew.% bis 10 Gew.%, bezogen auf die Gesamtmenge an eingesetztem HMF, bevorzugt beträgt der Anteil an HMF-Oligomer 0,1 Gew.% bis 8 Gew.%, bezogen auf die Gesamtmenge an eingesetztem HMF, besonders bevorzugt beträgt der Anteil an HMF-Oligomer 2 Gew.% bis 4 Gew.%, bezogen auf die Gesamtmenge an eingesetztem HMF. Es reichen bereits geringe Mengen an HMF-Oligomer aus, um unter den erfindungsgemäßen Bedingungen Phenolharze bereitzustellen, die eine zu Phenol-Formaldehyd-Harzen vergleichbare Bindungsstärke aufweisen. Es ist für den Fachmann selbstverständlich, dass auch höhere Anteile an HMF-Oligomer eingesetzt werden können. Ebenfalls von der Erfindung umfasst ist, dass das HMF-Oligomer bis zu oder bis nahezu 100 Gew. %, bezogen auf die Gesamtmenge an eingesetztem HMF, ausmacht.

Nach einer weiteren vorteilhaften Ausgestaltung des Verfahrens weist das HMF-Oligomer 2 bis 20 Einheiten auf, bevorzugt 2 bis 10 Einheiten, besonders bevorzugt 2 bis 4 Einheiten. HMF-Oligomere mit 2 bis 10 Einheiten sind unter moderaten Bedingungen, das heißt bei Raumtemperatur und unter Normaldruck, gut wasserlöslich, sodass die HMF-Oligomere problemlos für eine Polykondensation in einem wässrigen Medium eingesetzt werden können. HMF-Oligomere mit 2 bis 4 Einheiten weisen eine verbesserte Wasserlöslichkeit auf. HMF-Oligomere mit 2 Einheiten sind besonders gut wasserlöslich.

Bei der polykondensationsfähigen phenolischen Verbindung kann es sich um solche handeln, die üblicherweise für die Herstellung von thermisch härtbaren Phenolharzen verwendet werden.

Als polykondensationsfähige phenolische Verbindung kommen hierbei prinzipiell alle hydroxylgruppentragenden aromatischen Verbindungen in Frage, die im Aromaten mindestens ein Kohlenstoffatom aufweisen, das für eine nukleophile Additionsreaktion zwischen phenolischer Verbindung und dem HMF geeignet ist.

Vorteilhafterweise ist die polykondensationsfähige phenolische Verbindung Phenol, Lignin, eine aus Lignin abgeleitete phenolische Verbindung, Resorcin, Hydrochinon, Hydroxyhydrochinon, Brenzcatechin, Phloroglucin oder ein Gemisch von mindestens zwei dieser Verbindungen.

Dabei können neben den genannten Komponenten auch noch weitere phenolische Verbindungen und/oder Aminoplastbildner vorhanden sein. Geeignete Aminoplastbildner sind Harnstoff, Melamin, substituiertes Melamin, substituierter Harnstoff, Acetylendiharnstoff, Guanidin, Thioharnstoff, Thioharnstoffderivat, Diaminoalkan, Diamidoalkan oder ein Gemisch von mindestens zwei dieser Aminoplastbildner.

Nach einer weiteren bevorzugten Ausgestaltung des Verfahrens ist das für die Polykondensation eingesetzte HMF-Oligomer ein kohlenstoffverknüpftes HMF-Oligomer.

HMF-Oligomere werden im Sinne der vorliegenden Erfindung als kohlenstoffverknüpfte HMF-Oligomere bezeichnet, sofern zumindest zwei HMF-Einheiten über eine Kohlenstoff-Kohlenstoff-Bindung unter Einbeziehung eines aromatisch gebundenen Kohlenstoffatoms an Position 3 oder 4 des Furanrings einer der beiden HMF-Einheiten verknüpft sind. Insbesondere enthält das kohlenstoffverknüpfte HMF-Oligomer zumindest eine erste Einheit, deren Aldehydgruppen-Kohlenstoffatom mit einem aromatisch gebundenen Kohlenstoffatom des Furanrings einer zweiten Einheit verknüpft ist.

Die Erfinder haben herausgefunden, dass sich neben HMF-Oligomeren, die aus der Verknüpfung von Aldehyd- und/oder Hydroxylgruppen der HMF-Einheiten resultieren und die die entsprechenden Ether-, Hemiacetal- und/oder Acetal-Bindungen aufweisen, sowohl unter sauren als auch unter basischen Bedingungen HMF-Oligomere bilden, bei denen Einheiten über eine Kohlenstoff-Kohlenstoff-Bindung verknüpft sind. Diese Bindungen können beispielsweise bei einem elektrophilen Angriff einer Aldehydgruppe eines ersten HMF-Monomers oder einer HMF-Einheit eines HMF-Oligomers auf das Kohlenstoffatom in Position 3 oder 4 eines Furanrings eines zweiten HMF-Monomers oder einer HMF-Einheit eines HMF-Oligomers entstehen.

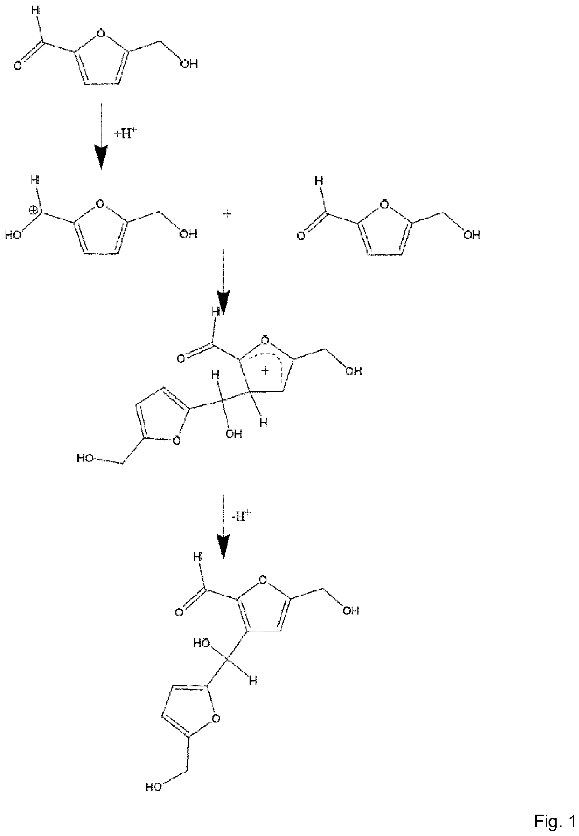

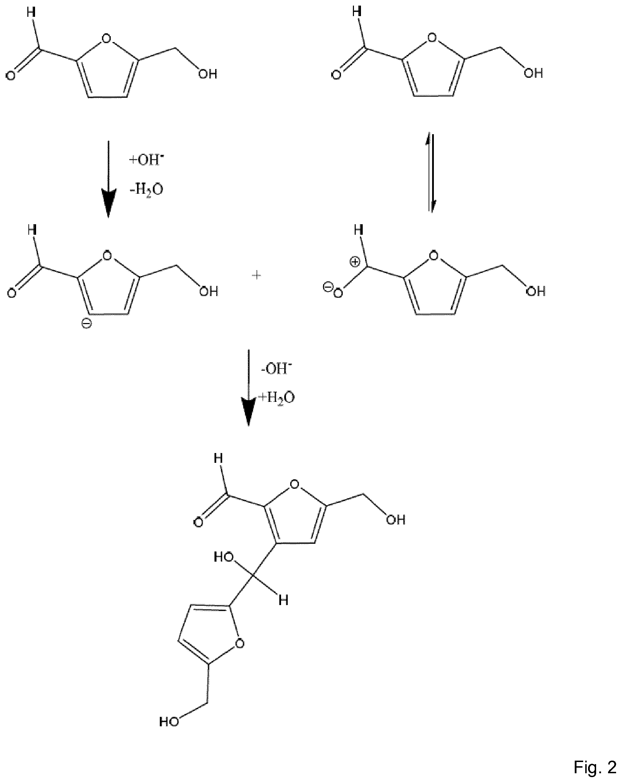

Die für die HMF-Oligomerbildung im Sauren und im Basischen vorgeschlagenen Mechanismen können den Figuren 1 und 2 entnommen werden. Aus diesen wird unter anderem ersichtlich, dass HMF-Oligomere, die über eine Verknüpfung durch eine Kohlenstoff-Kohlenstoff-Bindung verfügen, gleichzeitig auch über mehr freie funktionelle Aldehyd- und/oder Hydroxyl-Gruppen verfügen als HMF-Oligomere, bei denen die Verbindung lediglich über Aldehyd- und/ Hydroxylgruppen des HMFs zustande kommt. Hierdurch ergeben sich sehr reaktive HMF-Oligomere, die über zusätzliche Vernetzungsmöglichkeiten verfügen.

In dem kohlenstoffverknüpften HMF-Oligomer können neben der unter Einbeziehung eines aromatisch gebundenen Kohlenstoffs geknüpften Bindung auch andere Bindungen wie Ether-, Hemiacetal-, und/oder Acetal-Bindungen enthalten sein. Zur Erhöhung der Reaktivität des resultierenden Phenolharzes ist es ausreichend, wenn bereits zwei der HMF-Einheiten unter Einbeziehung eines aromatisch gebundenen Kohlenstoffatoms verknüpft sind. Insbesondere enthalten kohlenstoffverknüpfte HMF-Oligomere mit 2 Einheiten einen vergleichsweise hohen Anteil an freien funktionellen, reaktiven Gruppen pro HMF-Oligomer. Das kohlenstoffverknüpfte HMF-Oligomer kann auch mehrere solcher Kohlenstoff-Kohlenstoff-Verknüpfungen aufweisen.

Ferner können neben den kohlenstoffverknüpften HMF-Oligomeren noch weitere HMF-Oligomere mit Ether-, Hemiacetal- und/oder Acetal-Bindungen enthalten sein. Aufgrund des hohen Anteils an freien funktionellen Gruppen reichen bereits geringe Mengen an kohlenstoffverknüpftem HMF-Oligomer aus, um sehr reaktive Oligomere bereitzustellen. Ebenfalls von der Erfindung umfasst ist, dass das kohlenstoffverknüpfte HMF-Oligomer bis zu oder bis nahezu 100 Gew.%, bezogen auf die Gesamtmenge an HMF-Oligomer, ausmacht.

Nach einer weiteren vorteilhaften Ausgestaltung des Verfahrens enthält das Verfahren mindestens einen weiteren Schritt, der 5-Hydroxymethylfurfural, welches mindestens ein HMF-Oligomer umfasst, für den Umsetzungsschritt bereitstellt.

Bevorzugt enthält der Bereitstellungsschritt, dass man eine Lösung, enthaltend HMF-Monomere und/oder HMF-Oligomere, Bedingungen aussetzt, die zur Bildung von HMF-Oligomeren führen. Die Erfinder haben festgestellt, dass wässrige HMF-Lösungen, die beispielsweise aus kristallinem HMF mit Wasser hergestellt wurden, unter Bildung der HMF-Oligomere altern. Die Menge und das Molekulargewicht der HMF-Oligomere können hierbei über dem Fachmann geläufige Analysemittel wie HPLC und NMR-Spektroskopie bestimmt werden.

Die Bildung von HMF-Oligomeren unter moderaten Bedingungen, das bedeutet, unter Normaldruck und Raumtemperatur, kann im Bereich von Stunden, Tagen oder Wochen liegen.

Besonders bevorzugt umfassen die Bedingungen, denen die HMF-Lösung ausgesetzt wird, eine Alkalisierung oder Ansäuerung der Lösung. Ebenfalls besonders bevorzugt umfassen die Bedingungen das Erhitzen der Lösung, gegebenenfalls in Kombination mit einer Ansäuerung oder Alkalisierung, und/oder ein Lösungsmittelentzug, wie er beispielsweise mittels Rotationsverdampfer bei Unterdruck zur Konzentrierung durchgeführt werden kann. Durch Ansäuerung, Alkalisierung, Konzentrieren und Erhitzen kann der Alterungsprozess beschleunigt werden.

Eine besonders bevorzugte Variante des Bereitstellungsschritts beinhaltet, dass man 5-Hydroxymethylfurfural, welches mindestens ein HMF-Oligomer umfasst, durch Behandlung einer wässrigen Suspension von cellulosehaltiger Biomasse und/oder einer wässrigen Kohlenhydrat-Lösung von mindestens einer Hexose und/oder einer wässrigen 5-Hydroxymethylfurfural-Lösung unter hydrothermalen Bedingungen bereitstellt.

Die Behandlung von Biomasse wie pflanzlichen Rohstoffen, von Kohlenhydraten oder von aus Kohlenhydraten abgeleiteten Verbindungen unter hydrothermalen Bedingungen zur Gewinnung von 5-HMF(-Monomeren) ist bekannt und sieht vor, das Ausgangsmaterial in wässrigem Medium Druck und erhöhter Temperatur auszusetzen. Bei der Behandlung einer wässrigen Suspension von cellulosehaltiger Biomasse und/oder einer wässrigen Kohlenhydrat-Lösung von mindestens einer Hexose und/oder einer wässrigen 5-Hydroxymethylfurfural-Lösung unter hydrothermalen Bedingungen werden HMF-Oligomere gebildet.

Cellulosehaltige Biomasse, die häufig als Abfallprodukt der landwirtschaftlichen Erzeuger anfällt, ist aufgrund Ihres geringen Kostenfaktors besonders bevorzugt. Bevorzugte Hexosen sind Fructose oder Glucose, insbesondere kann es sich um Fructose oder Gemische aus Fructose und Glucose handeln.

Bevorzugte hydrothermale Bedingungen sind Sattdampfdruck und Temperaturen von 150 bis 250°C. Dies hat den Vorteil, dass die Bildung von HMF-Oligomeren in Abhängigkeit des Ausgangsmaterials innerhalb von Minuten bis zu wenigen Stunden abgeschlossen ist.

Bevorzugt wird der Bereitstellungsschritt so lange durchgeführt, bis die gewünschte Menge an HMF-Oligomer erreicht ist oder die Reaktion abgeschlossen ist. Es ist für den Fachmann selbstverständlich, dass die verschiedenen Varianten der Bereitstellung von HMF, welches mindestens ein HMF-Oligomer umfasst, miteinander kombiniert werden können, um zum gewünschten Ergebnis zu gelangen. Beispielsweise kann der Bereitstellungsschritt eine Behandlung unter hydrothermalen Bedingungen sowie eine Konzentrierung durch Lösungsmittelentzug enthalten.

Bevorzugt liegt das HMF, welches mindestens ein HMF-Oligomer umfasst, am Ende des Bereitstellungsschritts in wässriger Lösung vor. Es sind jedoch auch andere Formen des HMF geeignet, beispielsweise nach vollständigem Lösungsmittelentzug.

Weiter bevorzugt ist es, den Gehalt, den Anteil an Oligomer, bezogen auf die HMF-Gesamtmenge, die Größe und/oder die Konzentration des Oligomers oder der Oligomere zu beeinflussen. Besonders bevorzugt wird der Anteil und/oder Gehalt des Oligomers oder der Oligomere beeinflusst, indem die im Beschaffungsschritt erhaltene Lösung einer Filtration an mindestens einem Filtriermittel unterzogen wird. Die Behandlung einer wässrigen HMF-Lösung nach einer hydrothermalen Karbonisierung wird beispielsweise in der DE 10 2014 112 240 A1 beschrieben.

Besonders bevorzugt liegt das HMF, welches mindestens ein HMF-Oligomer umfasst, am Ende des Bereitstellungsschritts in wässriger Lösung in einer Konzentration von 25 Gew.% bis 80 Gew.% vor. Noch weiter bevorzugt in einer Konzentration von 27 Gew.% bis 75 Gew.%, weiter besonders bevorzugt in einer Konzentration von 27 Gew.% bis 73 Gew.%.

Gemäß einem weiteren Aspekt betrifft die vorliegende Erfindung ein thermisch härtbares Phenolharz, das durch das oben beschriebene Verfahren erhältlich ist.

Bevorzugt umfasst das thermisch härtbare Phenolharz wenigstens ein durch Polykondensation von phenolischen Verbindungen mit 5-Hydroxymethylfurfural (HMF) erhaltenes Polymer, wobei das Polymer ein Polykondensationsprodukt einer phenolischen Verbindung mit einem HMF-Oligomer ist.

Unter dem Begriff Polymer werden im Sinne der vorliegenden Erfindung Produkte der Polykondensation verstanden. Die Polymere sind üblicherweise wasserunlöslich.

Hinsichtlich bevorzugter phenolischer Verbindungen und gegebenenfalls zusätzlicher Aminoplastbildner kann auf die oben gemachten Angaben verwiesen werden.

Der Feststoffgehalt des Phenolharzes kann über einen breiten Bereich variiert werden. Der Feststoffgehalt beträgt wenigstens 35 Gew.%. Bevorzugt liegt der Feststoffgehalt des Phenolharzes im Bereich von 40 bis 80 Gew.%, weiter bevorzugt im Bereich von 40 bis 75 Gew.%, besonders bevorzugt zwischen 40 und 70 Gew.%.

Bevorzugt beträgt das molare Verhältnis der HMF-Gesamtmenge zu der Gesamtmenge an phenolischer Verbindung im Phenolharz 0,5:1 bis 4:1, bevorzugt beträgt das molare Verhältnis 1,3:1 bis 3:1, weiter bevorzugt beträgt das molare Verhältnis 1:1 bis 2,7:1, besonders bevorzugt beträgt das molare Verhältnis 1,5:1 bis 2,6:1, weiter besonders bevorzugt 2:1 bis 2,5:1.

Nach einer bevorzugten Ausgestaltung des Phenolharzes ist das Polymer ein Polykondensationsprodukt einer phenolischen Verbindung mit einem kohlenstoffverknüpften HMF-Oligomer, das zumindest eine erste HMF-Einheit enthält, die mit einem aromatisch gebundenen Kohlenstoff einer zweiten HMF-Einheit verknüpft ist.

Hinsichtlich der kohlenstoffverknüpften HMF-Oligomere kann auf die oben gemachten Angaben verwiesen werden.

Die Phenolharze eignen sich insbesondere zur Herstellung von Verbundwerkstoffen aus lignocellulosehaltigem Material wie Holzspänen, Holzfasern oder Holzchips. Die Herstellung der Holzverbundwerkstoffe erfolgt nach den auf diesem Fachgebiet allgemein bekannten Methoden. Die Holzverbundwerkstoffe werden erhalten, indem man das lignocellulosehaltige Material mit den Phenolharzen in Kontakt bringt und die Phenolharze anschließend aushärtet, was mit einer Vernetzung einhergeht.

Die Aushärtung wird bevorzugt vorgenommen, indem man das mit dem lignocellulosehaltigen Material versehene Phenolharz verpresst. Üblicherweise kommen Drücke von 1 bis 30 mPa zum Einsatz. In der Regel erfolgt die Verpressung bei einer Temperatur im Bereich von 120°C bis 250°C. Die konkrete Temperatur kann hierbei in Abhängigkeit des Phenolharzes, des lignocellulosehaltigen Materials und der gewünschten Eigenschaften der Verbundwerkstoffe gewählt werden. Aufgrund der Reaktivität der Phenolharze genügen bereits wenige Minuten zum Erhalt von Holzwerkstoffen mit guten mechanischen Eigenschaften. Bevorzugt liegt die Pressdauer im Bereich von 3 bis 10 Minuten, besonders bevorzugt liegt die Pressdauer im Bereich von 5 bis 8 Minuten. Eine kurze Pressdauer ist sowohl aus produktionstechnischen als auch wirtschaftlichen Gesichtspunkten vorteilhaft.

Vorteilhafterweise kann bei den Phenolharzen vollkommen auf einen Härter verzichtet werden.

Die erhaltenen Holzverbundwerkstoffe können schließlich zur Stabilisierung in einem Trockenschrank oder Holztrockner bei Temperaturen im Bereich von 10°C bis 100°C unter kontrollierter Atmosphäre nachbehandelt werden. Eine solche kann beispielsweise eine relative Luftfeuchte im Bereich von 40% bis 70% umfassen.

Ein weiterer Vorteil in der Herstellung eines Holzverbundwerkstoffs mit thermisch härtbaren Phenolharzen ist, dass die Holzverbundwerkstoffe formaldehyd-frei und auf Basis natürlicher, erneuerbarer Rohstoffe hergestellt werden können und dabei eine sehr gute Beständigkeit gegen Feuchtigkeit, insbesondere Wasserdampf, aufweisen. Noch ein weiterer Vorteil ist, dass aufgrund der Reaktivität der Phenolharze kurze Presszeiten im Minutenbereich ausreichen, um einen Holzverbundwerkstoff mit sehr guten mechanischen Eigenschaften zu erhalten.

Die Phenolharze eignen sich insbesondere zur Verwendung für die Herstellung von Sperrholz-, Holzfaserverbund-, Span- oder Mehrschichtplatten.

Ein weiterer Vorteil der Phenolharze besteht darin, dass diese eine zu Phenol-Formaldehyd-Harzen vergleichbare Bindungsstärke aufweisen.

Weitere Vorteile und vorteilhafte Ausgestaltungen sind den Ansprüchen und der nachfolgenden Zeichnung zu entnehmen.

Es zeigen
- Figur 1: einen vorgeschlagenen Mechanismus der KohlenstoffKohlenstoff-Bindungsbildung unter sauren Bedingungen anhand der Dimerisierung zweier HMF-Moleküle, sowie
- Figur 2: einen vorgeschlagenen Mechanismus der KohlenstoffKohlenstoff-Bindungsbildung unter basischen Bedingungen anhand der Dimerisierung zweier HMFMoleküle.

Sämtliche Merkmale der Erfindung können sowohl einzeln als auch in beliebiger Kombination miteinander erfindungswesentlich sein.

## Patentansprüche

1. Verfahren zur Herstellung von thermisch härtbaren Phenolharzen enthaltend den Schritt der Umsetzung einer polykondensationsfähigen phenolischen Verbindung mit 5-Hydroxymethylfurfural (HMF) unter zur Bildung von Polykondensationsprodukten führenden Bedingungen, **dadurch gekennzeichnet, dass** das HMF mindestens ein HMF-Oligomer umfasst, und der Umsetzungsschritt bei pH-Werten von über 7 für mehr als 60 Minuten durchgeführt wird.

2. Verfahren nach Anspruch 1, **dadurch gekennzeichnet, dass** der Umsetzungsschritt bei pH-Werten von 7,5 bis 14 durchgeführt wird.

3. Verfahren nach Anspruch 1 oder 2, **dadurch gekennzeichnet, dass** der Umsetzungsschritt für mehr als 60 Minuten bis 15 Stunden durchgeführt wird.

4. Verfahren nach einem der vorhergehenden Ansprüche, **dadurch gekennzeichnet, dass** der Umsetzungsschritt bei Temperaturen im Bereich von 40°C bis 170°C durchgeführt wird.

5. Verfahren nach einem der vorhergehenden Ansprüche, **dadurch gekennzeichnet, dass** der Umsetzungsschritt in mindestens zwei Stufen vorgenommen wird, wobei eine erste Stufe des Umsetzungsschritts bei Temperaturen im Bereich von 40°C bis 80°C durchgeführt wird, und eine weitere Stufe des Umsetzungsschritts bei Temperaturen durchgeführt wird, die höher als die Temperaturen der ersten Stufe sind.

6. Verfahren nach einem der vorhergehenden Ansprüche, **dadurch gekennzeichnet, dass** der Umsetzungsschritt in einer ersten Stufe für 50 bis 100 Minuten bei Temperaturen im Bereich von 40°C bis 80°C und in einer zweiten Stufe für 15 Minuten bis 14 Stunden bei Temperaturen im Bereich von 85°C bis 170°C durchgeführt wird.

7. Verfahren nach einem der vorhergehenden Ansprüche, **dadurch gekennzeichnet, dass** das molare Verhältnis der eingesetzten HMF-Menge zu der Gesamtmenge an phenolischer Verbindung 0,5:1 bis 4:1 beträgt, wobei das molare Verhältnis ermittelt wird, indem die molare Masse von HMF als Berechnungsgrundlage dient, ohne Berücksichtigung, welcher Anteil der HMF-Menge tatsächlich als Monomer vorliegt oder in HMF-Oligomere eingebunden ist.

8. Verfahren nach einem der vorhergehenden Ansprüche, **dadurch gekennzeichnet, dass** der Anteil an HMF-Oligomer, bezogen auf die Gesamtmenge an eingesetztem HMF, 0,05 Gew.% bis 10 Gew.% beträgt.

9. Verfahren nach einem der vorhergehenden Ansprüche, **dadurch gekennzeichnet, dass** das HMF-Oligomer 2 bis 20 Einheiten aufweist.

10. Verfahren nach einem der vorhergehenden Ansprüche, **dadurch gekennzeichnet, dass** die polykondensationsfähige phenolische Verbindung Phenol, Lignin, eine aus Lignin abgeleitete phenolische Verbindung, Resorcin, Hydrochinon, Hydroxyhydrochinon, Brenzcatechin, Phloroglucin oder ein Gemisch von mindestens zwei dieser Verbindungen ist

11. Verfahren nach einem der vorhergehenden Ansprüche, **dadurch gekennzeichnet, dass** das HMF-Oligomer ein kohlenstoffverknüpftes HMF-Oligomer ist.

12. Verfahren nach einem der vorhergehenden Ansprüche, **dadurch gekennzeichnet, dass** das Verfahren mindestens einen weiteren Schritt enthält, der 5-Hydroxymethylfurfural, welches mindestens ein HMF-Oligomer umfasst, für den Umsetzungsschritt bereitstellt.

13. Verfahren nach Anspruch 12, **dadurch gekennzeichnet, dass** das 5-Hydroxymethylfurfural durch Behandlung einer wässrigen Suspension von cellulosehaltiger Biomasse und/oder einer wässrigen Kohlenhydrat-Lösung von mindestens einer Hexose und/oder einer wässrigen 5-Hydroxymethylfurfural-Lösung unter hydrothermalen Bedingungen bereitgestellt wird.

## Claims

1. Method for the preparation of thermally curable phenolic resins, comprising the step of reacting a polycondensable phenolic compound with 5-hydroxymethylfurfural (HMF) under conditions leading to the formation of polycondensation products, **characterized in that** the HMF comprises at least one HMF oligomer, and the reaction step is carried out at pH values above 7 for more than 60 minutes.

2. Method according to claim 1, **characterized in that** the reaction step is carried out at pH values of 7.5 to 14.

3. Method according to claim 1 or 2, **characterized in that** the reaction step is carried out for more than 60 minutes to 15 hours.

4. Method according to any one of the preceding claims, **characterized in that** the reaction step is carried out at temperatures in the range of 40°C to 170°C.

5. Method according to any one of the preceding claims, **characterized in that** the reaction step is carried out in at least two stages, a first stage of the reaction step being carried out at temperatures in the range of 40°C to 80°C, and a further stage of the reaction step being carried out at temperatures higher than the temperatures of the first stage.

6. Method according to any one of the preceding claims, **characterized in that** the reaction step is carried out in a first stage for 50 to 100 minutes at temperatures in the range of 40°C to 80°C and in a second stage for 15 minutes to 14 hours at temperatures in the range of 85°C to 170°C.

7. Method according to any one of the preceding claims, **characterized in that** the molar ratio of the amount of HMF used to the total amount of phenolic compound is 0.5:1 to 4:1, the molar ratio being determined by using the molar mass of HMF as the basis for calculation, without taking into account which proportion of the amount of HMF is actually present as a monomer or is incorporated into HMF oligomers.

8. Method according to any one of the preceding claims, **characterized in that** the proportion of HMF oligomer, based on the total amount of HMF used, is 0.05% by weight to 10% by weight.

9. Method according to any one of the preceding claims, **characterized in that** the HMF oligomer has 2 to 20 units.

10. Method according to any one of the preceding claims, **characterized in that** the polycondensable phenolic compound is phenol, lignin, a phenolic compound derived from lignin, resorcinol, hydroquinone, hydroxyhydroquinone, pyrocatechol, phloroglucinol or a mixture of at least two of these compounds.

11. Method of any one of the preceding claims, **characterized in that** the HMF oligomer is a carbon-linked HMF oligomer.

12. Method according to any one of the preceding claims, **characterized in that** the method comprises at least one further step of providing 5-hydroxymethylfurfural comprising at least one HMF oligomer for the reaction step.

13. Method according to claim 12, **characterized in that** the 5-hydroxymethylfurfural is provided by treating an aqueous suspension of cellulosic biomass and/or an aqueous carbohydrate solution of at least one hexose and/or an aqueous 5-hydroxymethylfurfural solution under hydrothermal conditions.

## Revendications

1. Procédé de fabrication de résines phénoliques thermodurcissables comprenant l'étape de réaction d'un composé phénolique susceptible de polycondensation avec le 5-hydroxyméthylfurfural (HMF) dans des conditions conduisant à la formation de produits de polycondensation, **caractérisé en ce que** le HMF comprend au moins un oligomère HMF, et l'étape de réaction est mis en œuvre à des valeurs de pH supérieures à 7 pendant plus de 60 minutes.

2. Procédé selon la revendication 1, **caractérisé en ce que** l'étape de réaction est mis en œuvre à des valeurs de pH de 7,5 à 14.

3. Procédé selon la revendication 1 ou 2, **caractérisé en ce que** l'étape de réaction est mis en œuvre pendant plus de 60 minutes à 15 heures.

4. Procédé selon l'une quelconque des revendications précédentes, **caractérisé en ce que** l'étape de réaction est mis en œuvre à des températures dans une plage comprise entre 40 °C et 170 °C.

5. Procédé selon l'une quelconque des revendications précédentes, **caractérisé en ce que** l'étape de réaction est mis en œuvre en au moins deux étages, où un premier étage de l'étape de réaction est mis en œuvre à des températures dans une plage comprise entre de 40 °C et 80 °C, et un étage supplémentaire de l'étape de réaction est mis en œuvre à des températures, lesquelles sont supérieures aux températures du premier étage.

6. Procédé selon l'une quelconque des revendications précédentes, **caractérisé en ce que** l'étape de réaction est mis en œuvre lors d'un premier étage à des températures dans une plage comprise entre 40 °C et 80 °C pendant 50 à 100 minutes et lors d'un second étage à des températures dans une plage comprise entre 85 °C et 170 °C pendant 15 minutes à 14 heures.

7. Procédé selon l'une quelconque des revendications précédentes, **caractérisé en ce que** le rapport molaire entre la quantité de HMF utilisée et la quantité totale de composé phénolique est de 0,5:1 à 4:1, où le rapport molaire est déterminé **en ce que** la masse molaire de HMF sert de base de calcul, quelle que soit la fraction de la quantité de HMF réellement présente sous forme de monomère ou incorporée dans les oligomères de HMF.

8. Procédé selon l'une quelconque des revendications précédentes, **caractérisé en ce que** la fraction d'oligomères HMF, par rapport à la quantité totale de HMF utilisé, est de 0,05 % en poids à 10 % en poids.

9. Procédé selon l'une quelconque des revendications précédentes, **caractérisé en ce que** l'oligomère HMF comporte 2 à 20 motifs.

10. Procédé selon l'une quelconque des revendications précédentes, **caractérisé en ce que** le composé phénolique susceptible de polycondensation est le phénol, la lignine, un composé phénolique dérivé de la lignine, le résorcinol, l'hydroquinone, l'hydroxyhydroquinone, le pyrocatéchol, le phloroglucinol ou un mélange d'au moins deux dsdits composés.

11. Procédé selon l'une quelconque des revendications précédentes, **caractérisé en ce que** l'oligomère de HMF est un oligomère de HMF lié au carbone.

12. Procédé selon l'une quelconque des revendications précédentes, **caractérisé en ce que** le procédé comprend au moins une étape supplémentaire,laquelle fournit le 5-hydroxyméthylfurfural, lequel comprend au moins un oligomère de HMF, pour l'étape de réaction.

13. Procédé selon la revendication 12, **caractérisé en ce que** le 5-hydroxyméthylfurfural est fourni par traitement d'une suspension aqueuse de biomasse cellulosique et/ou d'une solution aqueuse d'hydrates de carbone d'au moins un hexose et/ou d'une solution aqueuse de 5-hydroxyméthylfurfural dans des conditions hydrothermales.
